# EUROPEAN PATENT APPLICATION

(11) **EP 2 390 839 A1**
(43) Date of publication of application: **30.11.2011**
(21) Application number: 11150636.6
(22) Date of filing: 11.01.2011
(51) Int. Cl.: G06T 7/00, A61B 8/08, G01S 15/89

(54) **3D ultrasound apparatus and method for operating the same**

(30) Priority: 31.05.2010 KR 20100051144
(71) Applicant: Samsung Medison Co., Ltd., Nam-myun Hongchun-gun Kangwon do (KR)
(72) Inventor: Lee, Kwang Hee, 302-743, Daejeion (KR)
(74) Representative: Schmid, Wolfgang

(57) **Abstract**

A 3-dimensional (3D) ultrasound apparatus and a method for operating the same are provided. A 3D ultrasound apparatus includes a first processor (105), a second processor (107) and a controller (113). The first processor determines a start point from an image data obtained by scanning an object in a human body. The second processor extracts a top image of the object from the image data based on the start point. The controller controls a sagittal view of the object by rotating the image data, using the top image.

## Description

### CROSS-REFERENCE TO RELATED APPLICATION

This application claims the benefit of Korean Patent Application No. 10-2010-0051144, filed on May 31, 2010, in the Korean Intellectual Property Office, the disclosure of which is incorporated herein by reference.

### BACKGROUND

### 1. Field of the Invention

The present invention relates to a 3-dimensional (3D) ultrasound apparatus and a method for operating the same, in which a top image of an object in a human body is extracted from an image data based on a start point in the image data obtained by scanning the object, and the image data is rotated using the extracted top image, thereby automatically determining a sagittal view of the object.

### 2. Description of the Related Art

An ultrasound system is an apparatus that irradiates an ultrasound signal from a surface of a human body towards a target part, that is, an object such as a fetus, an internal organ, and the like, under the body surface and obtains an image of a monolayer or blood flow in soft tissue from information in the reflected ultrasound signal. The ultrasound system has been widely used together with other image diagnostic systems such as X-ray diagnostic systems, computerized tomography (CT) scanners, magnetic resonance image (MRI) systems and nuclear medicine diagnostic systems because of its various merits such as a small size, a low price, real-time image display, and high stability through elimination of any radiation exposure.

Also, a general method for diagnosing a Down's syndrome fetus is to measure the thickness of a fetus' nuchal translucency (NT) through the ultrasound system. The method was developed by Nicolaides in 1992, and it has been known that in a case where a fetus has an abnormal symptom, body fluid is accumulated in subcutaneous tissues at the nape of a fetus' neck and therefore, the fetus' NT of the fetus becomes thick.

Another method for diagnosing a Down's syndrome fetus is to measure the angle between a fetus' maxilla and nasal bone, that is, the frontmaxillary facial (FMF) angle, and the like. For example, in a case where the FMF angle of a fetus is greater than 88.7 degrees as compared with 78.1 that is the FMF angle of a normal fetus, it is highly likely that the fetus has Down's syndrome.

Therefore, in order to easily diagnose a Down's syndrome fetus, it is required to more easily and precisely inspect the thickness of the fetus' NT or the angle between the fetus' maxilla and nasal bone.

However, since a measured value changes depending on the position or angle of a fetus, the sagittal view for the fetus should be precisely controlled so as to properly inspect the thickness of the fetus' NT or the angle between the fetus' maxilla and the fetus' nasal bone.

Accordingly, it is required to develop a technology for precisely controlling the sagittal view for a fetus.

### SUMMARY

An aspect of the present invention provides a 3-dimensional (3D) ultrasound apparatus and a method for operating the same, in which a top image of an object in a human body is extracted from an image data based on a start point in the image data obtained by scanning the object, and the image data is rotated using the extracted top image, thereby automatically determining a sagittal view of the object.

An aspect of the present invention also provides a 3D ultrasound apparatus and a method for operating the same, in which, in a case where an object in a human body is a fetus, a top image of the object corresponding to a basic data on the rotation of an image data is easily extracted from the image data obtained by scanning the object, using the direction of the fetus' head, thereby rapidly controlling a sagittal view of the object.

According to an aspect of the present invention, there is provided a 3D ultrasound apparatus, the apparatus including a first processor to determine a start point from an image data obtained by scanning an object in a human body, a second processor to extract a top image of the object from the image data based on the start point, and a controller to control a sagittal view of the object by rotating the image data, using the top image.

According to an aspect of the present invention, there is provided a method for operating a 3D ultrasound apparatus, the method including determining a start point from an image data obtained by scanning an object in a human body, extracting a top image of the object from the image data based on the start point, and rotating the image data using the top image, thereby controlling a sagittal view of the object.

### BRIEF DESCRIPTION OF THE DRAWINGS

These and/or other aspects, features, and advantages of the invention will become apparent and more readily appreciated from the following description of exemplary embodiments, taken in conjunction with the accompanying drawings of which:
- FIG. 1: is a block diagram illustrating a configuration of a 3-dimensional (3D) ultrasound apparatus according to an embodiment of the present invention;
- FIG. 2: is a diagram illustrating an example of an image for each direction with respect to an object extracted in a 3D ultrasound apparatus according to the embodiment of the present invention;
- FIG. 3: is a diagram illustrating a method for determining a start point from an image data obtained by scanning an object, using a 3D ultrasound apparatus according to the embodiment of the present invention;
- FIG. 4: is a diagram illustrating a method for determining the direction of an object, using a 3D ultrasound apparatus according to the embodiment of the present invention;
- FIG. 5: is a diagram illustrating an example of extracting a top image for an object as a basic data for controlling a sagittal view, using a 3D ultrasound apparatus according to the embodiment of the present invention;
- FIG. 6: is a diagram illustrating an example of correcting a front image for an object, using a 3D ultrasound apparatus according to the embodiment of the present invention;
- FIG. 7: is a diagram illustrating an example of controlling a sagittal view by rotating an image data for an object, using a 3D ultrasound apparatus according to the embodiment of the present invention; and
- FIG. 8: is a flowchart illustrating a method for operating a 3D ultrasound apparatus according to an embodiment of the present invention.

### DETAILED DESCRIPTION

Reference will now be made in detail to exemplary embodiments of the present invention, examples of which are illustrated in the accompanying drawings, wherein like reference numerals refer to the like elements throughout. Exemplary embodiments are described below to explain the present invention by referring to the figures.

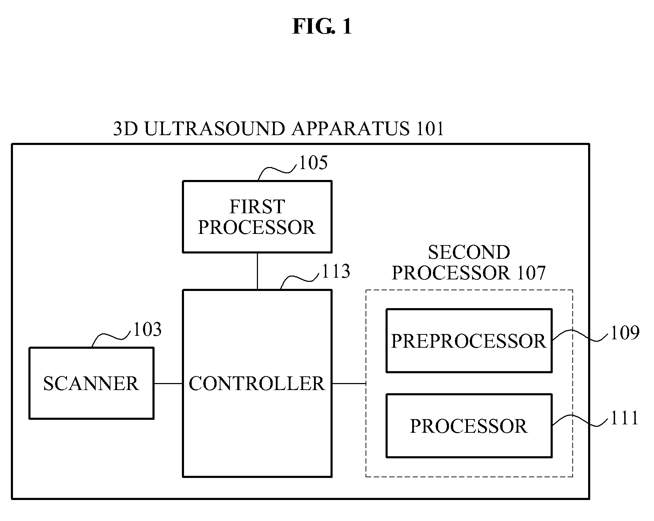

FIG. 1 is a block diagram illustrating a configuration of a 3-dimensional (3D) ultrasound apparatus 101 according to an embodiment of the present invention.

Referring to FIG. 1, the 3D ultrasound apparatus 101 includes a scanner 103, a first processor 105, a second processor 107, and a controller 113.

The scanner 103 may extract at least one of a side image (A-plane in a side direction), a top image (B-plane in a top direction) and a front image (C-plane in a front direction) with respect to an object in a human body from an image data obtained by scanning the object and then may display the at least one of the side image, the top image, and the front image on a screen. In this instance, the scanner 103 may remove noise from each of the side image, the top image, and the front image so that the contours of images with respect to the object are clearly displayed on the screen.

The first processor 105 determines a start point from the image data obtained by scanning an object in a human body. Here, the object in the human body may include a fetus, an internal organ, and the like. In a case where the object is a fetus, the first processor 105 may extract a side image of the fetus from the image data and identify the fetus' nasal bone. Then, the first processor 105 may determine a start point using the fetus' nasal bone.

Specifically, the first processor 105 may extract a side image of the fetus from the image data and identify the fetus' nasal bone or maxilla by using the intensity of the side image. In this instance, the first processor 105 may place a seed at the fetus' nuchal translucency (NT) and set a window area based on the seed. Then, the first processor 105 may identify a part in the window area of which the intensity is highest as the fetus' nasal bone or maxilla while moving the window area upward. The intensity being the highest is a result of bone being reflected most strongly and thus, an area in which the bone is placed appears most bright.

The first processor 105 may identify the frontmaxillary facial (FMF) angle between the fetus' nasal bone and maxilla, using the fetus' nasal bone and maxilla.

Subsequently, the first processor 105 may determine a virtual point as the start point. Here, the virtual point is spaced apart upward by a selected distance, for example, 1.3 cm to 1.5 cm, from a point at which a vertical line that passes through an end point of the fetus' nasal bone is intersected with a horizontal line that passes through the fetus' NT.

The second processor 107 extracts a top image of the object from the image data based on the start point. Specifically, the second processor includes a preprocessor 109 and a processor 111.

In a case where the object is a fetus, the preprocessor 109 may determine the direction of the fetus' head. For example, the preprocessor 109 moves a first virtual plane in a side direction at a predetermined interval in a direction perpendicular to the first virtual plane in the side direction with respect to the image data, thereby extracting a plurality of image data included in the first virtual plane. Subsequently, the preprocessor 109 may identify the direction of the FMF angle between the fetus' nasal bone and maxilla from the plurality of image data included in the first virtual plane. In a case where an amount of image data including an FMF angle in a first direction, for example, a left direction, is greater than an amount of image data including an FMF angle in a second direction, for example, a right direction, the preprocessor 109 may determine the first direction as the direction of the fetus' head.

The processor 111 moves a second virtual plane in a top direction at a predetermined interval in the direction of the fetus' head from the start point with respect to the image data, thereby extracting a plurality of image data included in the second virtual plane.

Subsequently, the processor 111 may extract any one of the plurality of image data included in the second virtual plane as the top image. In this instance, the processor 111 may measure the outer circumferences of images from the image data included in the second virtual plane and may select each image data having a larger circumference than the mean of the measured outer circumferences for all image data. Then, the processor 111 may extract, as the top image, an image data having the smallest template matching among each of the image data having a larger circumference than the mean of the measured outer circumferences.

For example, in a case where the object is a fetus, the processor 111 may measure the circumferences of ellipses corresponding to the fetus' head from the plurality of image data included in the second virtual plane, and extract, as the top image, an image data having the smallest template matching among image data each having a larger circumference than the mean of the measured circumferences of the ellipses. In this instance, the processor 111 may extract an image data having the circumference of an ellipse, relatively highly matched to a selected template, for example, an ellipse having an occipitofrontal diameter (OFD) of 2.5 cm and an aspect ratio of 1.5, as the top image.

Accordingly, the processor 111 moves the second virtual plane in the direction of the fetus' head from the start point, so as to more rapidly extract the top image as compared with a case that the second virtual plane is moved with respect to the entire fetus from the fetus' tiptoe to head.

The controller 113 may identify the fetus' nasal bone by using the intensity of the side image of the fetus, extracted from the image data, and may move one side of the image data in front and vertical directions so that the fetus' nasal bone is placed at the highest position. In this instance, the controller 113 controls the image of the fetus not to be diagonally placed by moving the one side of the image data in the front and vertical directions so that the fetus' nasal bone is placed at the highest position. Accordingly, the image of the fetus can be placed bilaterally symmetric in the front image of the fetus.

The controller 113 may control a sagittal view of the object by rotating the image data using the top image. In this instance, the controller 113 may pass through an arbitrary point in the second virtual plane in the top direction and rotate the image data with respect to a virtual axis that passes through the side image.

Thus, the controller 113 rotates the image data, using the intensity of an image included in the side image or the left/right matching of the appearance of an image included in the top image, thereby automatically controlling the sagittal view of the object.

### 1) Rotation of image data using falx area

In a case where the object is a fetus, the controller 113 may extract a side image of the fetus from the image data and rotate the image data so that the brightness intensity in a falx area of the fetus, included in the side image, is largest.

Here, where the side image is a mid-sagittal, a part of the fetus, that is, the falx area is uniformly distributed bright. Conversely, where the side image is not a mid-sagittal, the falx area is not uniformly bright, and a dark area appears.

Accordingly, using a characteristic of brightness as described above, the controller 113 may rotate the image data so that the falx area is most brightly and uniformly distributed while moving and rotating an ultrasound data with respect to the center of the fetus' head.

### 2) Rotation of image data using left/right matching degree

The controller 113 may automatically control a sagittal view of the object by matching a figure corresponding to the fetus included in the top image and rotating the image data so that the left/right matching of the matched figure is highest.

For example, in a case where the matched figure is an ellipse, the controller 113 may vertically place the major axis of the ellipse, and rotate the image data so that the left and right of the ellipse are most symmetric with respect to the major axis.

According to the present embodiment, a top image of an object is extracted from an image data based on a start point of the image data obtained by scanning the object in a human body, and the image data is rotated using the extracted top image, so that a sagittal view of the object can be automatically determined.

Also, in a case where the object in the human body is a fetus, a top image of the object corresponding to a basic data on the rotation of the image data from the image data obtained by scanning the object, using the direction of the fetus' head, so that the sagittal view of the object can be rapidly controlled.

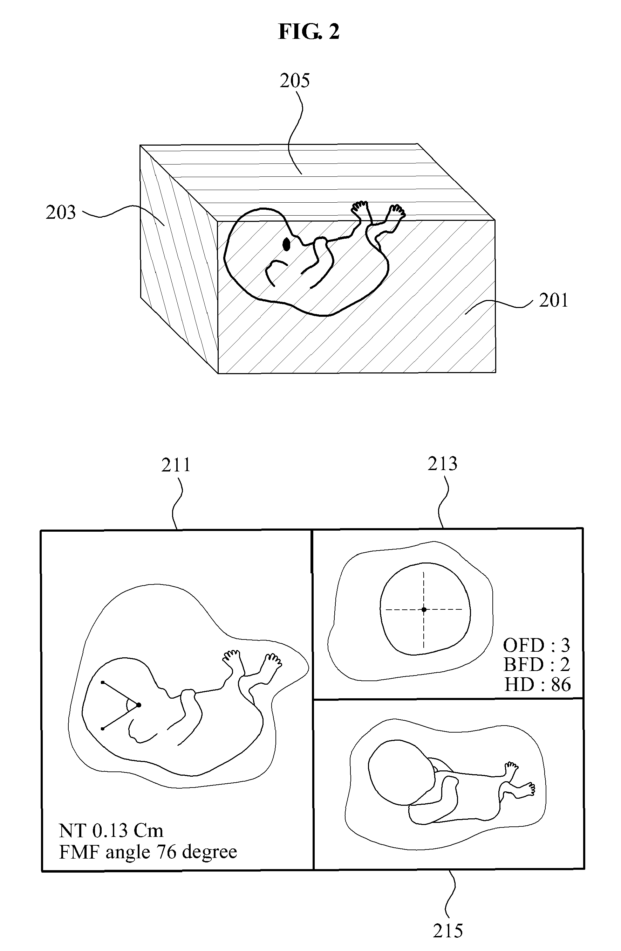

FIG. 2 is a diagram illustrating an example of an image for each direction with respect to an object extracted in a 3D ultrasound apparatus according to the embodiment of the present invention.

Referring to FIG. 2, the 3D ultrasound apparatus may extract a side image, a top image, or a front image from an image data obtained by scanning an object in a human body and may display the image on the screen.

For example, the 3D ultrasound apparatus may extract a side image in a side direction, which displays a 'first plane' 201, from an image data obtained by scanning an object and display the side image in a first area 211 on the screen. The 3D ultrasound apparatus may extract a top image in a top direction, which displays a 'second plane' 203, from the image data obtained by scanning the object, and may display the top image in a second area 213 on the screen. The 3D ultrasound apparatus may extract a front image in a front direction, which displays a 'third plane' 205, from the image data obtained by scanning the object, and may display the front image in a third area 215 on the screen.

As the image data is rotated or moved based on a selected reference, the 3D ultrasound apparatus updates the side image, the top image, or the front image and displays the updated image on the screen, thereby easily detecting a 3D object.

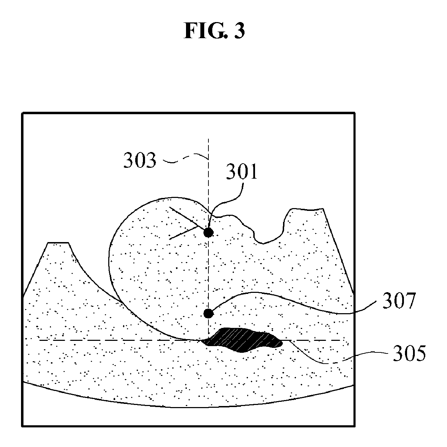

FIG. 3 is a diagram illustrating a method for determining a start point from an image data obtained by scanning an object, using a 3D ultrasound apparatus according to the embodiment of the present invention.

Referring to FIG. 3, the 3D ultrasound apparatus may extract a side image of a fetus from an image data obtained by scanning the fetus, and may identify the fetus' nasal bone or maxilla by using the intensity of the side image. For example, the 3D ultrasound apparatus may identify a part of the side image of which intensity is highest as the fetus' nasal bone or maxilla.

In this instance, the 3D ultrasound apparatus may determine a virtual point 307 as a start point. Here, the virtual point 307 is spaced apart upward by a selected distance from a point at which a vertical line 303 that passes through an end point 301 of the fetus' nasal bone is intersected with a horizontal line 305 that passes through fetus' NT.

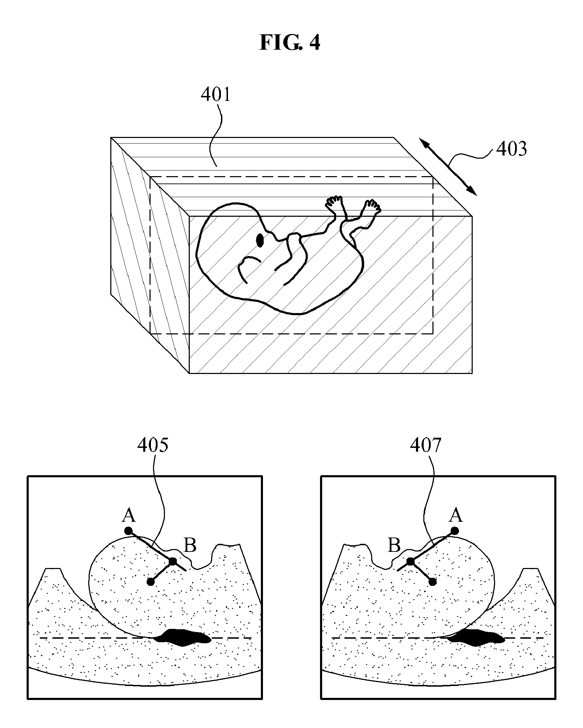

FIG. 4 is a diagram illustrating a method for determining the direction of an object, using a 3D ultrasound apparatus according to the embodiment of the present invention.

Referring to FIG. 4, in a case where the object is a fetus, the 3D ultrasound apparatus may determine the direction of fetus' head from an image data by scanning the fetus.

For example, the 3D ultrasound apparatus moves a first virtual plane 401 in a side direction in a direction 403 perpendicular to the first virtual plane 410 at a predetermined interval with respect to the image data, thereby extracting a plurality of image data included in the first virtual plane 401. In this instance, the 3D ultrasound apparatus may apply a top-hat transform to the image data so as to precisely and easily extract the fetus' nasal bone and maxilla.

Subsequently, the 3D ultrasound apparatus identifies the direction of the FMF angle between the fetus' nasal bone and maxilla from the plurality of image data included in the first virtual plane 401.

In a case where an amount of image data including an FMF angle 405 in a first direction, for example, a left direction, are greater than an amount of image data including an FMF angle 407 in a second direction, for example, a right direction, the 3D ultrasound apparatus may determine the first direction as the direction of the fetus' head. Specifically, the 3D ultrasound apparatus may provide grades as 'left: right = 7 : 3' with respect to the direction of the fetus' head, estimated based on the plurality of image data. Finally, the 3D ultrasound apparatus may determine the direction of the fetus' head as the left direction to which a relatively high grade is provided.

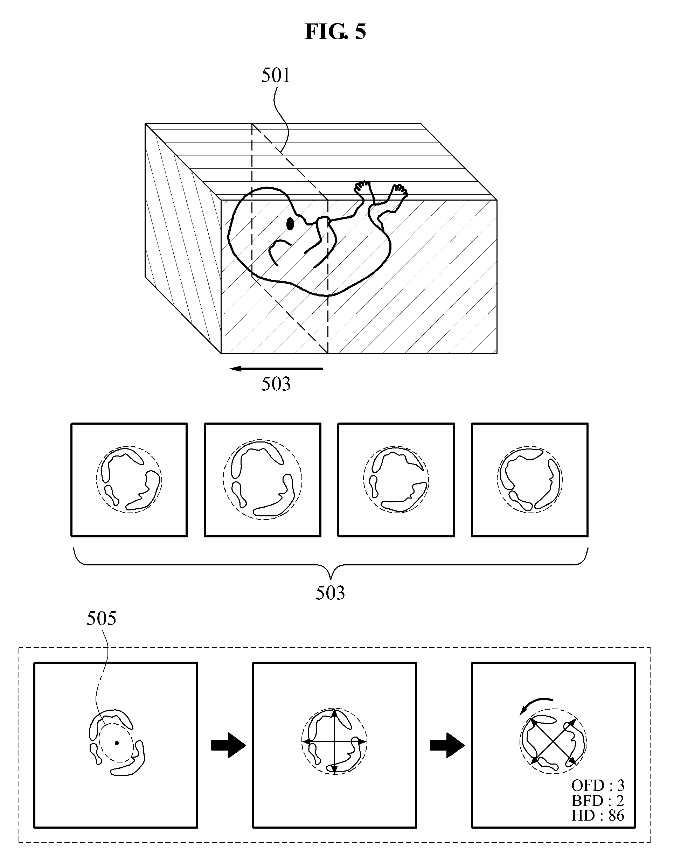

FIG. 5 is a diagram illustrating an example of extracting a top image for an object as a basic data for controlling a sagittal view, using the 3D ultrasound apparatus according to the embodiment of the present invention.

Referring to FIG. 5, the 3D ultrasound apparatus moves a second virtual plane 501 in a top direction in the direction 503 of fetus' head at a predetermined interval from a start point 501 with an image data obtained by scanning a fetus, thereby extracting a plurality of image data included in the second virtual plane 501.

Subsequently, the 3D ultrasound apparatus measures the circumferences of ellipses corresponding to the fetus' head from the image data included in the second virtual plane 501, and determines an image data having a larger circumference than the mean of the measured circumferences of the ellipses. For example, in a case where the number of image data included in the second virtual plane 501 is 10, the 3D ultrasound apparatus may determine four image data each having a larger circumference than the mean of the circumferences of ellipses, that is, 8.6 cm.

The 3D ultrasound apparatus may extract, as a top image, an image data having the smallest template matching among the image data each having a larger circumference than the mean of the circumferences of the ellipses. For example, the 3D ultrasonic apparatus may extract, as the top image, one image data having a circumference highly matched to a selected template, for example, an ellipse having an OFD of 2.5 cm and an aspect ratio of 1.5, among the four image data each having a larger circumference than the mean of the circumferences of the ellipses, that is, 8.6 cm.

Here, the 3D ultrasound apparatus may display an ellipse template 505 on an ellipse corresponding to the fetus' head in each of the image data, and may change the biparietal diameter (BPD) or OFD of the ellipse template 505 so that the ellipse template 505 is matched to the ellipse corresponding to the fetus' head. In this instance, the 3D ultrasound apparatus may extract an image data most highly matched to the ellipse template 505 by minimizing the change of the ellipse template 505.

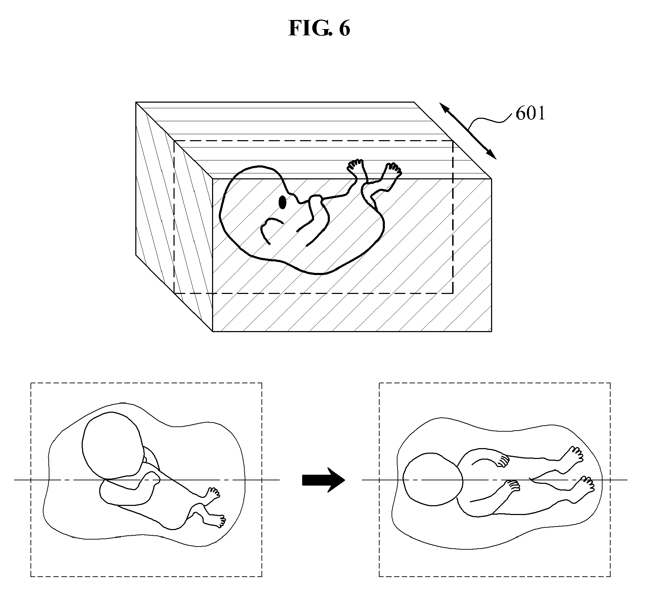

FIG. 6 is a diagram illustrating an example of correcting a front image for an object, using a 3D ultrasound apparatus according to the embodiment of the present invention.

Referring to FIG. 6, the 3D ultrasound apparatus may extract a side image of a fetus from an image data obtained by scanning the fetus, and move one side of the image data in a direction 601 perpendicular to the side image so that the fetus' nasal bone is placed at the highest position in the side image.

In this instance, the 3D ultrasound apparatus controls the image of the fetus not to be diagonally placed by moving the one side of the image data in the direction 601 perpendicular to the side image so that the fetus' nasal bone is placed at the highest position. Accordingly, the 3D ultrasound apparatus may display a front image so that the fetus is placed bilaterally symmetric in the front image of the fetus. That is, the 3D ultrasound apparatus may display the front image so that the fetus' face, arms, and legs are placed bilaterally symmetric in the front image.

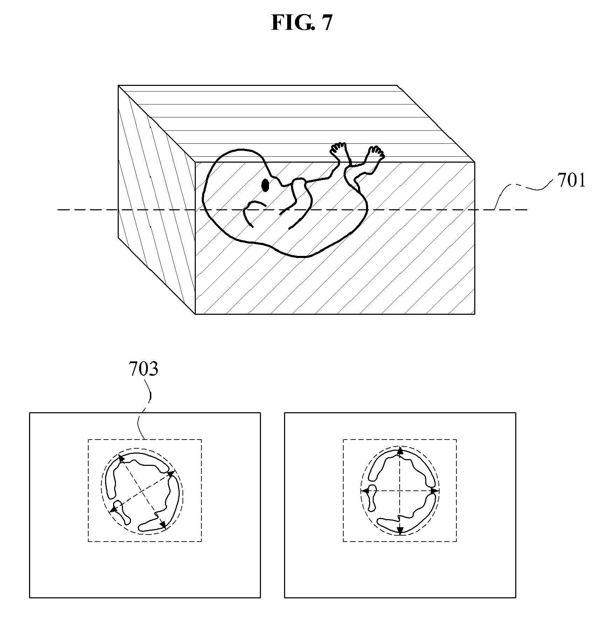

FIG. 7 is a diagram illustrating an example of controlling a sagittal view by rotating an image data for an object, using a 3D ultrasound apparatus according to the embodiment of the present invention.

Referring to FIG. 7, the 3D ultrasound apparatus extracts a top image of an object from an image data obtained by scanning the object and rotates the image data using the top image, thereby controlling a sagittal view of the object.

For example, the 3D ultrasound apparatus may automatically control a sagittal view of the object by setting a window area 703 in the top image, and by matching a figure corresponding to the object included in the top image and rotating the image data so that the left/right matching of the matched figure is highest. That is, in a case where the figure is an ellipse, the 3D ultrasound apparatus may vertically place the major axis of the ellipse and rotate the image data so that the left and right of the ellipse are most symmetric with respect to the major axis.

In a case where the ellipse of the top image is inclined, the 3D ultrasound apparatus controls the ellipse of the top image not to be inclined by rotating the image data with respect to a virtual axis 701 that passes through an arbitrary point in the second virtual plane in the top direction and passes the side image. Accordingly, the left/right matching of the circumference of the ellipse may be increased.

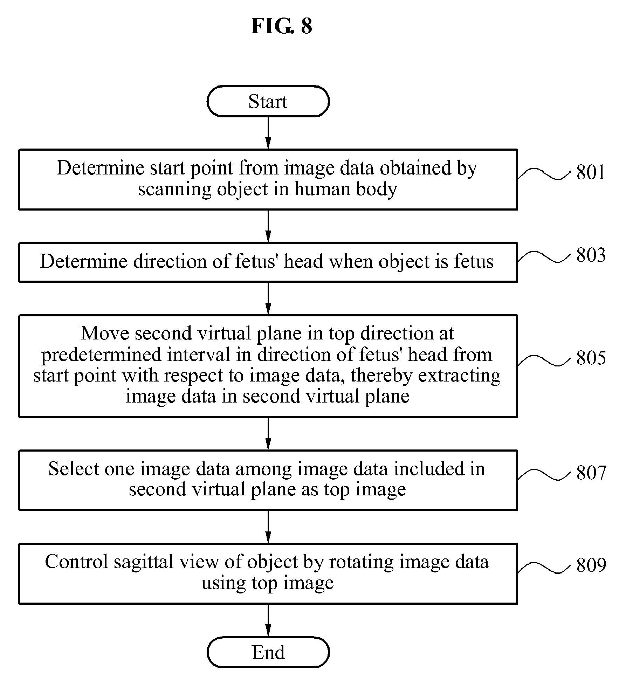

FIG. 8 is a flowchart illustrating a method for operating a 3D ultrasound apparatus according to an embodiment of the present invention.

Referring to FIG. 8, in operation 801, the 3D ultrasound apparatus determines a start point from an image data obtained by scanning an object in a human body.

In a case where the object is a fetus, the 3D ultrasound apparatus may extract a side image of the fetus from the image data and identify the fetus' nasal bone. Then, the 3D ultrasound apparatus may determine a start point using the fetus' nasal bone.

First, the 3D ultrasound apparatus may extract a side image of the fetus from the image data and identify fetus' nasal bone or maxilla by using the intensity of the side image. As bone is reflected most strongly, an area in which the bone is placed appears most bright. For this reason, the 3D ultrasound apparatus may identify a part of the side image of which intensity is highest as the fetus' nasal bone or maxilla.

Subsequently, the 3D ultrasound apparatus may determine a virtual point as the start point. Here, the virtual point is spaced apart upward by a selected distance, for example, 1.3 cm to 1.5 cm, from a point at which a vertical line that passes through an end point of the fetus' nasal bone is intersected with a horizontal line that passes through the fetus' NT.

In operation 803, in a case where the object is a fetus, the 3D ultrasound apparatus determines the direction of fetus' head.

For example, the 3D ultrasound apparatus moves a first virtual plane in a side direction at a predetermined interval in a direction perpendicular to the first virtual plane with respect to the image data, thereby extracting a plurality of image data included in the first virtual plane.

Subsequently, the 3D ultrasound apparatus may identify the direction of the FMF angle between the fetus' nasal bone and maxilla from the plurality of image data included in the first virtual plane. In a case where an amount of image data including an FMF angle in a first direction, for example, a left direction, are greater than an amount of image data including an FMF angle in a second direction, for example, a right direction, the 3D ultrasound apparatus may determine the first direction as the direction of the fetus' head.

In operation 805, the 3D ultrasound apparatus moves a second virtual plane in a top direction at a predetermined interval in the direction of the fetus' head from the start point with respect to the image data, thereby extracting a plurality of image data included in the second virtual plane.

In operation 807, the 3D ultrasound apparatus selects one image data among the image data included in the second virtual plane as the top image.

In this instance, the 3D ultrasound apparatus measures outer circumferences of an image from the image data included in the second virtual plane and calculates the mean of the measured outer circumferences. The 3D ultrasound apparatus may select each image data having a larger circumference than the mean of the measured outer circumferences for all image data and extract, as the top image, an image data having the smallest template matching among the image data each having a larger circumference than the mean of the outer circumferences.

In a case where the fetus is diagonally placed, the 3D ultrasound apparatus may move the image data using the fetus' nasal bone in the side image of the fetus. That is, the 3D ultrasound apparatus may control the fetus to be placed bilaterally symmetric in the front image of the fetus by moving one side of the image data in a direction perpendicular to the side image so that the fetus' nasal bone is placed at the highest position.

In operation 809, the 3D ultrasound apparatus controls a sagittal view of the object by rotating the image data using the top image. In this instance, the 3D ultrasound apparatus may pass through an arbitrary point in the second virtual plane in the top direction and rotate the image data with respect to a virtual axis that passes through the side image.

Specifically, in a case where the object is a fetus, the 3D ultrasound apparatus may control a sagittal view of the object by extracting a side image of the fetus from the image data and by rotating the image data so that the brightness intensity in a falx area of the fetus, included in the side image, is largest.

Alternatively, in a case where the object is a fetus, the 3D ultrasound apparatus may control a sagittal view of the object by matching a figure corresponding to the fetus included in a top image and rotating the image data so that the left/right matching of the matched figure is highest.

According to embodiments of the present invention, a top image of an object in a human body is extracted from an image data based on a start point in the image data obtained by scanning the object, and the image data is rotated using the extracted top image, thereby automatically determining a sagittal view of the object.

According to embodiments of the present invention, in a case where an object in a human body is a fetus, a top image of the object corresponding to a basic data on the rotation of an image data is easily extracted from the image data obtained by scanning the object, using the direction of the fetus' head, thereby rapidly controlling a sagittal view of the object.

The above-described exemplary embodiments of the present invention may be recorded in non-transitory computer-readable media including program instructions to implement various operations embodied by a computer. The media may also include, alone or in combination with the program instructions, data files, data structures, and the like. Examples of non-transitory computer-readable media include magnetic media such as hard disks, floppy disks, and magnetic tape; optical media such as CD ROM disks and DVDs; magneto-optical media such as optical disks; and hardware devices that are specially configured to store and perform program instructions, such as read-only memory (ROM), random access memory (RAM), flash memory, and the like. Examples of program instructions include both machine code, such as produced by a compiler, and files containing higher level code that may be executed by the computer using an interpreter.

Although a few exemplary embodiments of the present invention have been shown and described, the present invention is not limited to the described exemplary embodiments. Instead, it would be appreciated by those skilled in the art that changes may be made to these exemplary embodiments without departing from the principles and spirit of the invention, the scope of which is defined by the claims and their equivalents.

## Claims

1. A 3-dimensional (3D) ultrasound apparatus, the apparatus comprising:
a first processor to determine a start point from an image data obtained by scanning an object in a human body;
a second processor to extract a top image of the object from the image data based on the start point; and
a controller to control a sagittal view of the object by rotating the image data, using the top image.

2. The apparatus of claim 1, wherein, when the object is a fetus, the first processor identifies fetus' nasal bone by using the intensity of a side image of the fetus, extracted from the image data, and determines, as the start point, a virtual point spaced apart at a selected distance from a point at which a vertical line that passes through an end of the fetus' nasal bone is intersected with a horizontal line that passes through the fetus' nuchal translucency (NT).

3. The apparatus of claim 1, wherein, when the object is a fetus, the second processor determines the direction of the fetus' head.

4. The apparatus of claim 3, wherein:
the preprocessor identifies the direction of a frontmaxillary facial (FMF) angle between the fetus' nasal bone and maxilla from the image data included in a first virtual plane in a side direction while moving the first virtual plane in the side direction at a predetermined interval in a direction perpendicular to the first virtual plane with respect to the image data; and
when an amount of image data including an FMF angle in a first direction is greater than an amount of image data including an FMF angle in a second direction, the preprocessor determines the first direction as the direction of the fetus' head.

5. The apparatus of claim 3, wherein the second processor extracts any one of image data included in a second virtual plane in a top direction as the top image while moving the second virtual plane in the top direction at a predetermined interval in the direction of the fetus' head from the start point with respect to the image data.

6. The apparatus of claim 3, wherein the second processor measures outer circumferences of an image from the data image included in the second virtual plane in the top direction, select each image data having a larger circumference than the mean of the measured outer circumferences for all image data, and extracts, as the top image, an image data having the smallest template matching among the determined image data.

7. The apparatus of claim 1, wherein, when the object is a fetus, the controller identifies the fetus' nasal bone by using the intensity of the side image of the fetus extracted from the image data, and moves one side of the image data in a direction perpendicular to the side image so that the fetus' nasal bone is placed at the highest position.

8. The apparatus of claim 1, wherein, when the object is a fetus, the controller extracts a side image of the fetus from the image data and rotates the image data so that the brightness intensity in a falx area of the fetus, included in the side image, is largest.

9. The apparatus of claim 1, wherein, when the object is a fetus, the controller matches a figure to the fetus included in the top image and rotates the image data so that a left/right matching of the matched figure is highest.

10. A method for operating a 3D ultrasound apparatus, the method comprising:
determining a start point from an image data obtained by scanning an object in a human body;
extracting a top image of the object from the image data based on the start point; and
rotating the image data using the top image, thereby controlling a sagittal view of the object.

11. The method of claim 10, wherein, when the object is a fetus, the determining of the start point comprises:
identifying the fetus' nasal bone by using the intensity of a side image of the fetus,
extracted from the image data; and
determining, as the start point, a virtual point spaced apart at a selected distance from a point at which a vertical line that passes through an end of the fetus' nasal bone is intersected with a horizontal line that passes through fetus' NT.

12. The method of claim 10, wherein, when the object is a fetus, the extracting of the top image of the object comprises determining the direction of the fetus' head.

13. The method of claim 10, wherein, when the object is a fetus, the extracting of the top image for the object comprises:
identifying the direction of an FMF angle between the fetus' nasal bone and maxilla from the image data included in a first virtual plane in a side direction while moving the first virtual plane in the side direction at a predetermined interval in a direction perpendicular to the first virtual plane with respect to the image data; and
determining a first direction as the direction of the fetus' head when an amount of image data including an FMF angle in the first direction is greater than an amount of image data including an FMF angle in a second direction.

14. The method of claim 10, wherein, when the object is a fetus, the extracting of the top image of the object comprises extracting any one of image data included in a second virtual plane in a top direction as the top image while moving the second virtual plane in the top direction at a predetermined interval in the direction of the fetus' head from the start point with respect to the image data.

15. The method of claim 10, wherein, when the object is a fetus, the extracting of the top image for the object comprises:
measuring outer circumferences of an image from the data image included in the second virtual plane in the top direction;
determining image data each having a larger circumference than the mean of the measured outer circumferences; and
extracting, as the top image, an image data having the smallest template matching among the determined image data.
